# EUROPÄISCHE PATENTSCHRIFT

(11) **EP 0 440 879 B1**
(45) Veröffentlichungstag und Bekanntmachung des Hinweises auf die Patenterteilung: **20.12.1995**
(21) Anmeldenummer: 90117989.5
(22) Anmeldetag: 19.09.1990
(51) Int. Cl.: A61F 2/26

(54) **Implantierbare Penisprothese**
Implantable penile prosthesis
Prothèse pénienne implantable

(30) Priorität: 09.02.1990 DE 9001508 U
(43) Veröffentlichungstag der Anmeldung: 14.08.1991
(73) Patentinhaber: ESKA KUNSTSTOFFTECHNIK GMBH & CO., vormals Walter Koss, D-65366 Geisenheim (DE)
(72) Erfinder: Grundei, Hans, D-6222 Geisenheim (DE)
(74) Vertreter: Blumbach, Kramer & Partner

(56) Entgegenhaltungen:
- DE-A- 3 446 157
- DE-A- 3 522 758

## Beschreibung

Die vorliegende Erfindung betrifft eine Penisprothese gemäß dem Oberbegriff des Anspruchs 1.

Neben stabförmigen Penisprothesen aus Kunststoff, die aufgrund einer Einlage aus Feinsilberdrähten in Form eines Zopfes in die jeweils gewünschte Form gebogen werden können (DE-U 83 35 133.7), sind auch stabförmige Penisprothese der eingangs genannten Art bekannt, die eine mit einer Flüssigkeit unter Druck auffüllbare Erektionskammer und einen mit der Erektionskammer in Verbindung stehenden Schlauchanschluß aufweisen. Im Ruhestand sind sie biegeweich und schlaff. Wenn mittels einer kleinen, ebenfalls implantierbaren Pumpe eine Erektionskammer in Form eines schlauchförmigen Hohlraums unter Flüssigkeitsdruck gesetzt wird, weitet sich die stabförmige Prothese auf und wird gleichzeitig steifer. Bei Druckentlastung durch Betätigung eines Ventils wird wieder der Ruhestand erreicht.

Als gattungsbildender Stand der Technik ist aus der DE-A 35 22 758 ferner eine unabhängige Penisprothese bekannt, bei der sowohl die Pumpe als auch ein Flüssigkeitsreservoir in der Prothese selbst angeordnet sind. Die Prothese weist zudem ein von Hand verformbares Entlastungsventil zur Entlüftung der Druckkammer auf. Diese besteht aus einer faltbaren, zylinderförmigen Hülle aus unelastischem Material, welches sich selbst unter Druck nicht ausdehnen oder strecken kann.

Der vorliegenden Erfindung liegt die Erkenntnis zugrunde, daß unter Nachahmung der natürlichen Vorgänge bei einer Erektion mittels der Prothese neben einer Versteifung, insbesondere auch eine Längenvergrößerung stattfinden muß, wobei jedoch bei höheren Flüssigkeitsdrücken eine unzulässig hohe Längenausdehnung der Prothese vermieden werden muß. Dies wird erfindungsgemäß dadurch erreicht, daß die Hülle in ihrer Länge ausdehnbar ist, wobei die Längenausdehnung mittels eines Fadens begrenzt wird, der den Vorderteil und den Hinterteil des Stabes miteinander verbindet. Als Verbindung zu einer mit einem separaten Flüssigkeitsreservoir verbundenen Pumpe weist der Stab ferner einen mit der Erektionskammer in Verbindung stehenden Schlauchanschluß auf. Wenn nun die Erektionskammer unter Druck mit Flüssigkeit gefüllt wird, dehnt sich die Hülle der Länge noch bis zu der durch den Faden vorgegebenen maximalen Ausdehnung hin aus, so daß die Prothese unter Nachahmung der natürlichen Vorgänge bei der Erektion insgesamt ebenfalls länger wird, wobei jedoch durch den Faden eine unzulässig hohe Längenausdehnung bei höheren Flüssigkeitsdrücken sicher vermieden wird.

Weiterbildungen der Erfindung sind Gegenstand der Unteransprüche. Die in ihrer Länge ausdehnbare Hülle ist zweckmäßig nach Art eines Faltenbalges ausgebildet. Hierfür stehen als bewährtes Material Gefäßprothesen zur Verfügung, die in großem Umfang für den Ersatz insbesondere von Venen im menschlichen Körper benutzt werden. Im Inneren der Hülle wird zur Verbesserung der Abdichtung in vorteilhafter Weise ein dehnbarer Abdichtschlauch für die Erektionskammer angeordnet.

Der vordere Teil des Stabes, also im implantierten Zustand der distale Teil, enthält zweckmäßig eine Versteifungseinlage. Diese kann in bekannter Weise aus verdrillten Feinsilberdrähten bestehen, die einen Zopf bilden. Zur Erhöhung der Bruchfestigkeit auch nach vielen Biegevorgängen können die einzelnen Drähte in bekannter Weise (DE-U-83 35 133) einzeln in einen Schrumpfschlauch eingebettet sein.

Zweckmäßigerweise wird der Faden, der beispielsweise aus Nylon bestehen kann, im vorderen Teil der Prothese an der Versteifungseinlage befestigt. Von hier aus führt er durch die Erektionskammer zu einer Verankerungsstelle im hinteren Teil.

Die Stabteile und die Hülle bestehen aus Silicon einer zum Implantieren zugelassenen Qualität. Die einzelnen Teile der Prothese sind miteinander verklebt.

Die Prothesen werden jeweils paarweise implantiert und in die Schwellkörper eingesetzt.

Nachfolgend wird die Erfindung anhand eines in der Zeichnung dargestellten Ausführungsbeispiels erläutert. Es zeigen:

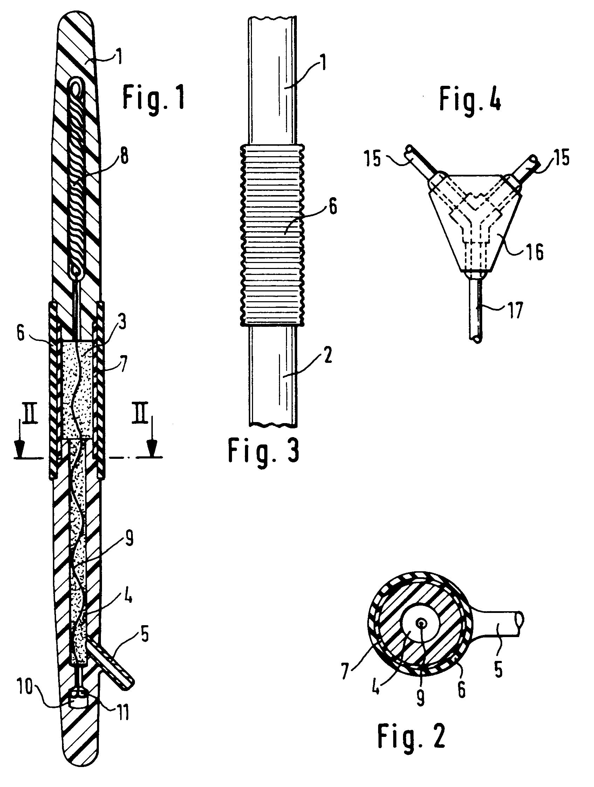

- Fig. 1: einen Längsschnitt durch eine Prothese als Ausführungsbeispiel der Erfindung;
- Fig. 2: einen vergrößerten Querschnitt der Prothese nach Fig. 1 entlang der Schnittlinie II-II;
- Fig. 3: eine Ansicht des mittleren Bereichs der Prothese nach Fig. 1 und 2;
- Fig. 4: ein Verteilerstück für die Verteilung der Druckflüssigkeit auf die beiden Prothesenstäbe eines Paares.

Die dargestellte Prothese weist einen vorderen (distalen) Teil 1 und einen hinteren (proximalen) Teil 2 auf, die aus Silicon hergestellt sind. Im mittleren Bereich der Prothese befindet sich eine Erektionskammer 3, die über eine Bohrung 4 im hinteren Teil 2 mit einem Schlauchanschluß 5 in Verbindung steht. Die Erektionskammer 3 wird nach außen durch eine Hülle in Form eines Faltenbalges 6 abgeschlossen, auf dessen Innenseite ein dünnwandiger Schlauch 7 zur besseren Abdichtung der Erektionskammer 3 angeordnet ist. Sowohl der Schlauch 7 als auch der Faltenbalg 6 sind mit dem vorderen Stabteil 1 und dem hinteren Stabteil 2 in angepaßten Ausnehmungen liegend verklebt.

Im vorderen Stabteil 1 ist ein verdrillter Zopf 8 aus Feinsilberdrähten angeordnet, die einzeln in Schrumpfschläuchen liegen. Der Zopf 8 bewirkt eine erwünschte Versteifung des vorderen Teils 1 und ermöglicht eine Formanpassung durch Verbiegen. Da die aus hochreinem Silber bestehenden Drähte des Zopfes 8 praktisch keine elastische Rückfederung aufweisen, behält der Stabteil 1 die jeweils gewählte Biegeform bei.

Am hinteren Ende des Zopfes 8 ist ein Nylonfaden 9 befestigt, der durch die Erektionskammer 3 und die Bohrung 4 zu einer Befestigungsstelle 10 im hinteren Stabteil 2 führt. Die Befestigungsstelle 10 wird durch eine kleine Kammer gebildet, in der ein Metallstück 11 zur Befestigung des Fadens 9 liegt. Statt eines gesonderten Metallteils 11 kann auch ein Knoten des Fadens 9 für die Festlegung in der Kammer 10 dienen.

Nach der Implantation von zwei Stäben werden zwei Schläuche (nicht gezeigt) auf die beiden Schlauchanschlüsse 5 aufgesteckt und zu den Anschlüssen 15 der Y-Verzweigung 16 (Fig. 4) geführt, die im Bereich der Peniswurzel unter die Haut implantiert wird. Vom Anschluß 17 der Verzweigung 16 führt dann ein weiterer Schlauch (nicht gezeigt) zu einer Pumpe (nicht gezeigt), die beispielsweise im Bereich der Leistenfurche unter die Haut implantiert ist. Die Pumpe kann in bekannter Weise eine durch Fingerdruck betätigte Pumpe mit einem Rückschlagventil und einem Reservoir für die Flüssigkeit, vorzugsweise physiologische Kochsalzlösung, sein. Wenn dann Flüssigkeit in die Erektionskammern 3 unter Druck gepumpt wird, dehnt sich der Faltenbalg 6 zusammen mit dem Schlauch 7 in Längsrichtung aus, so daß die Stäbe 1, 2 steifer und länger werden. Zusätzlich kann eine begrenzte Durchmesserzunahme im Bereich der Erektionskammer 3 und des unteren Stabteils 2 stattfinden.

Die Pumpe weist zweckmäßig ein Überdruckventil auf, um den maximal erzielbaren Druck zu begrenzen. Zusätzlich ist ein ebenfalls durch die Haut betätigbares Entlastungsventil vorgesehen, das ein Rückströmen der Flüssigkeit in das Pumpenreservoir ermöglicht. Anstelle einer handbetätigten Pumpe kann auch eine durch einen Elektromotor betätigte Pumpe Verwendung finden.

## Patentansprüche

1. Penisprothese in Form eines implantierbaren, aus Kunststoff hergestellten Stabes (1, 2), der eine mit einer Flüssigkeit ausfüllbare Erektionskammer (3) aufweist, die im mittleren Teil des Stabes (1, 2) angeordnet ist und aus einer schlauchförmigen Hülle (6) besteht, welche den vorderen (1) und den hinteren (2) Teil des Stabes (1, 2) miteinander verbindet,
dadurch gekennzeichnet,
daß die Hülle (6) in ihrer Länge ausdehnbar ist, wobei die Längenausdehnung mittels eines Fadens (9) begrenzt wird, der den vorderen (1) und den hinteren (2) Teil des Stabes (1, 2) miteinander verbindet,
und daß der Stab (1, 2) einen mit der Erektionskammer (3) in Verbindung stehenden Schlauchanschluß (5) für eine mit einem Flüssigkeitsreservoir verbundene Pumpe aufweist.

2. Penisprothese nach Anspruch 1,
dadurch gekennzeichnet,
daß die Hülle nach Art eines Faltenbalges (6) ausgebildet ist.

3. Penisprothese nach Anspruch 1 oder 2,
dadurch gekennzeichnet,
daß im Inneren der Hülle ein dehnbarer Abdichtschlauch (7) für die Erektionskammer (3) angeordnet ist.

4. Penisprothese nach einem der Ansprüche 1 bis 3,
dadurch gekennzeichnet,
daß im vorderen und/oder hinteren Teil (1) des Stabes eine Versteifungseinlage (8) eingebettet ist.

5. Penisprothese nach Anspruch 4,
dadurch gekennzeichnet,
daß die Versteifungseinlage (8) aus verdrillten Feinsilberdrähten besteht.

6. Penisprothese nach Anspruch 4 oder 5,
dadurch gekennzeichnet,
daß der Faden (9) im vorderen Teil (1) der Prothese an der Versteifungseinlage (8) befestigt ist und durch die Erektionskammer (3) zu einer Verankerungsstelle (10) im hinteren Teil führt.

7. Penisprothese nach einem der Ansprüche 3 bis 6,
dadurch gekennzeichnet,
daß die Stabteile (1, 2), die Hülle (6) und der Abdichtschlauch (7) miteinander verklebt sind.

## Claims

1. A penile prosthesis in the form of an implantable plastic rod (1, 2) comprising an erection chamber (3) fillable with a fluid and disposed in the central part of the rod (1, 2) and comprising a tubular sheath (6) which connects the front (1) to the rear (2) part of the rod (1, 2), characterised in that the sheath (6) is extendable in length, the length extension being limited by a thread (9) which connects the front (1) to the rear (2) part of the rod (1, 2), and the rod (1, 2) has a flexible-tube connection (5) connected to the erection chamber (3) and for a pump connected to a fluid reservoir.

2. A penile prosthesis according to claim 1, characterised in that the sheath is constructed like a bellows (6).

3. A penile prosthesis according to claim 1 or 2, characterised in that an extendable sealing tube (7) for the erection chamber (3) is disposed inside the sheath.

4. A penile prosthesis according to any of claims 1 to 3, characterised in that a stiffening insert (8) is embedded in the front and/or rear part (1) of the rod.

5. A penile prosthesis according to claim 4, characterised in that the stiffening insert (8) consists of twisted fine silver wires.

6. A penile prosthesis according to claim 4 or 5, characterised in that the thread (9) is secured to the stiffening insert (8) in the front part (1) of the prosthesis and extends through the erection chamber (3) to a place (10) where it is anchored in the rear part.

7. A penile prosthesis according to any of claims 3 to 6, characterised in that the rod parts (1, 2), the sheath (6) and the sealing tube (7) are stuck to one another.

## Revendications

1. Prothèse pénienne se présentant sous la forme d'une baguette (1, 2) implantable, fabriquée en plastique, qui présente une chambre d'érection (3) pouvant être remplie d'un liquide,qui est disposée dans la partie centrale de la baguette (1, 2) et comprend une enveloppe (6) en forme de tuyau, laquelle relie la partie avant (1) et la partie arrière (2) de la baguette (1, 2),
caractérisée en ce que l'enveloppe est extensible dans sa longueur, l'allongement longitudinal étant limité au moyen d'un fil (9) qui relie la partie avant (1) et la partie arrière (2) de la baguette (1, 2),
et en ce que la baguette (1, 2) présente un raccord de tuyau (5) communiquant avec la chambre d'érection (3) pour une pompe reliée à un réservoir de liquide.

2. Prothèse pénienne selon la revendication 1, caractérisée en ce que l'enveloppe est conçue à la façon d'un soufflet (6).

3. Prothèse pénienne selon la revendication 1 ou 2, caractérisée en ce qu'un tuyau étanche (7) extensible pour la chambre d'érection (3) est disposé à l'intérieur de l'enveloppe.

4. Prothèse pénienne selon l'une quelconque des revendications 1 à 3, caractérisée en ce qu'une couche intercalaire de renfort(8) est insérée dans la partie avant et/ou la partie arrière (1) de la baguette.

5. Prothèse pénienne selon la revendication 4, caractérisée en ce que la couche intercalaire de renfort (8) se compose de fils torsadés en argent fin.

6. Prothèse pénienne selon la revendication 4 ou 5, caractérisée en ce que le fil (9) est fixé dans la partie avant (1) de la prothèse sur la couche intercalaire de renfort (8), traverse la chambre d'érection (3) et conduit à un point de fixation (10) situé dans la partie arrière.

7. Prothèse pénienne selon l'une quelconque des revendications 3 à 6, caractérisée en ce que les parties de baguette (1, 2), l'enveloppe (6) et le tuyau étanche (7) sont reliés entre eux par collage.
